# EUROPÄISCHE PATENTSCHRIFT

(11) **EP 3 630 295 B1**
(45) Veröffentlichungstag und Bekanntmachung des Hinweises auf die Patenterteilung: **09.03.2022**
(21) Anmeldenummer: 18727233.1
(22) Anmeldetag: 18.05.2018
(51) Int. Cl.: A61Q 13/00, A61K 8/11, B01J 13/22, C11D 3/50, C11D 17/00

(54) **MIKROKAPSELSYSTEM FÜR POLYSENSORISCHE DUFTEFFEKTE I**
MICROCAPSULE SYSTEM FOR POLYSENSORY OLFACTORY EFFECTS I
SYSTÈME DE MICRO-CAPSULES POUR EFFETS PARFUMÉS POLYSENSORIELS

(30) Priorität: 24.05.2017 DE 102017111444
(43) Veröffentlichungstag der Anmeldung: 08.04.2020
(73) Patentinhaber: Henkel AG & Co. KGaA, 40589 Düsseldorf (DE)
(72) Erfinder: BAUER, Andreas, 41564 Kaarst (DE); HÄTZELT, André, 40591 Düsseldorf (DE); PESSEL, Frank, 40215 Düsseldorf (DE); GERIGK, Andreas, 41812 Erkelenz (DE); WILSCH-IRRGANG, Anneliese, 40593 Düsseldorf (DE); LAST, Klaus, 32423 Minden (DE); AMADO, Raul, 32423 Minden (DE)
(86) Internationale Anmeldenummer: PCT/EP2018/063147
(87) Internationale Veröffentlichungsnummer: WO 2018/215351

(56) Entgegenhaltungen:
- US-A- 4 891 172
- US-A1- 2014 178 442
- US-A1- 2016 177 241
- US-B1- 6 709 673

## Beschreibung

Die Erfindung liegt auf dem Gebiet der parfümhaltigen Mikrokapseln, sowie der kosmetischen Mittel, der Reinigungsmittel und Textilbehandlungsmittel, die parfümhaltige Mikrokapseln enthalten, und Verfahren zur Freisetzung von Duftstoffen aus diesen Mikrokapseln bei der Anwendung besagter Mittel.

Eine Vielzahl von kosmetischen Mitteln, Reinigungsmitteln und Textilbehandlungsmitteln enthalten empfindliche Inhaltsstoffe, wie z.B. Riechstoffe, ätherische Öle, Parfümöle und Pflegeöle, Farbstoffe oder antibakterielle Wirkstoffe. Nachteilig ist, dass solche Inhaltsstoffe, die in solchen Mitteln eingesetzt werden, häufig schon bei der Lagerung und/oder vor dem gewünschten Anwendungszeitpunkt ihre Aktivität verlieren oder zumindest stark reduziert werden und zwar beispielsweise durch chemische Reaktionen infolge Interaktion mit anderen Bestandteilen der jeweiligen Mittel und/oder durch physikalische Einflüsse.

Um solche Substanzen kontrolliert und am gewünschten Einsatzort mit maximaler Wirkung einzusetzen werden Aktiv-oder Wirksubstanzen wie Duftstoffe, Pflegeöle, antibakterielle Wirkstoffe und dergleichen den Produkten oftmals in räumlich abgegrenzter, geschützter Form zugesetzt. Häufig werden empfindliche Substanzen in Kapseln verschiedener Größen eingeschlossen, auf geeigneten Trägermaterialien adsorbiert oder chemisch modifiziert. Die Freisetzung kann dann mit Hilfe eines geeigneten Mechanismus erfolgen, beispielsweise mechanisch durch Scherung, oder diffusiv direkt aus dem Matrixmaterial.

Es existieren bereits zahlreiche kommerzielle Verkapselungssysteme, die auf natürlichen oder künstlichen Polymeren basieren. Diese können einen Wirkstoff oder dessen Lösung umschließen und dann in der Hülle physikalisch oder chemisch vernetzt werden oder durch einen Koazervationsprozess mit einem anderen Polymer ausgefällt werden. Aus dem Stand der Technik sind Mikrokapseln bekannt, die als Kernmaterial flüssige, feste oder gasförmige Stoffe enthalten können. Als Material für die Kapselwände sind beispielsweise Phenol-Formaldehyd-Polymere, Melamin-Formaldehyd-Polymere, Polyurethan, Gelatine, Polyamide oder Polyharnstoffe gebräuchlich. Kosmetische Mittel, Reinigungsmittel und Textilbehandlungsmittel, welche Mikrokapseln enthalten, sind als solche bekannt. Insbesondere haben sich Mikrokapseln aus Melamin-Formaldehyd-Harzen in diesen Mitteln bewährt, da diese besonders stabil sind.

So beschreibt die europäische Offenlegungsschrift EP 0 967 007 A2 ein Verfahren zur Mikroverkapselung fester, biologisch aktiver Substanzen, insbesondere Pestizide, durch Polykondensation eines Melamin- bzw. Phenol/Formaldehyd-Harzes oder eines Harnstoff/Formalin-Harzes in Dispersion in Gegenwart der jeweils zu verkapselnden Aktivsubstanz und eines nichtionischen polymeren Schutzkolloids zur Stabilisierung der Emulsion, wobei Mikrokapseln mit mittleren Teilchendurchmessern von 0,1 bis 300 um erhalten werden. Dieses Verfahren ist nur zur Verkapselung fester biologischer Aktivsubstanzen geeignet. Zur Stabilisierung der Emulsion muss der Emulsion ein polymeres Schutzkolloid zugesetzt werden. Es werden herkömmliche Kapselsysteme mit einfacher Hüllenstruktur beschrieben.

K. Hong "Melamine resin microcapsules containing fragrant oil : synthesis and characterization" in Materials Chemistry and Physics 58 (1999), Seiten 128-131 beschreiben die Herstellung von wirkstoffhaltigen Melaminharzmikrokapseln mit langer Lebensdauer, die ein Duftöl enthalten, durch insitu-Polymerisation von Migrinöl als Kapselkernmaterial, Melamin und Formalin als Kapselhüllenmaterial, Natriumlaurylsulfat als Emulgator und Polyvinylalkohol als Schutzkolloid. Es entstehen mit Duftöl beladene Kapselsysteme mit einfacher Hüllenstruktur.

In der U.S. Patentschrift 6,709,673 B1 wird ein Mikrokapselsystem zur Lagerung und Freisetzung von Aktivstoffen in Abhängigkeit bestimmter Umweltparameter offenbart, wobei eine Vielzahl von Kapseln von mindestens einer Zwischenkapsel und mindestens einer äußeren Kapsel eingeschlossen ist. Weiterhin offenbart werden Mikrokapselsysteme, in denen eine äußere Kapsel eine Vielzahl von inneren Kapseln einschließt, wobei die äußere Kapsel und die innere Kapsel unterschiedliche Aktivstoffe beinhalten. Dieses Beispiel betrifft äußere Kapseln, welche bei menschlicher Körpertemperatur aufbrechen und über einen gewissen Zeitraum einen ersten Aktivstoff, d.h. eine relativ milde chemische Substanz zur Neutralisierung menschlichen Schweißes, freisetzen, sowie innere Kapseln, welche reibungsbedingt eine ähnliche, jedoch hochkonzentrierte Substanz freisetzen.

Die Patentveröffentlichung US 2016/0177241 A1 beschreibt eine Textilkonditionierzusammensetzung umfassend zwei unterschiedliche Arten von Kapseln, wobei sich die Kapseln hinsichtlich ihrer Kapselwände unterschieden, welche zwar aus ein und demselben Polymer, allerdings mit verschiedenen Härtungstemperaturen und/oder -zeiten hergestellt wurden.

Die Patentveröffentlichung US 2014/0178442 A1 offenbart Zusammensetzungen umfassend einen in der Zusammensetzung dispergierten "parent" Duftstsoff und Mikrokapseln enthaltend einen "nonparent" Duftstoff. Die Kapselwände umfassen ein Polyacrylatmaterial und ermöglichen eine reibungsgesteuerte Freisetzung des Duftstoffs.

Die U.S. Patentschrift 4,891,172 offenbart ein Verfahren zur Herstellung von sogenannten Doppelkapseln mittels Dispergieren einer Dispersion von Mikrokapseln, enthaltend eine hydrophile oder eine hydrophobe Substanz als Kernsubstanz, in einer hydrophoben Substanz.

Die bekannten Kapselsysteme ermöglichen allerdings nicht die Erzeugung verschiedener Duftprofile über den kompletten Anwendungszyklus eines Produktes. Dies kann insbesondere dann wünschenswert oder vorteilhaft sein, wenn sich der Dufteindruck beim Verbraucher über die Zeit ändern soll. Denkbar sind hier vor allem ein erster Dufteindruck, der für das Produkt oder dessen Einsatzzweck charakteristisch ist und ggf. auch einen bestimmten Wiedererkennungswert bewirkt, beispielsweise einen überwiegend kosmetischen Geruchseindruck beim Öffnen oder der Anwendung des Produkts, welcher nach der Anwendung durch einen davon unterschiedlichen Geruchseindruck, beispielsweise einen vorwiegend fruchtigen Geruchseindruck, abgelöst wird. Damit wäre es beispielsweise möglich den Geruch eines Wasch- und Reinigungsmittelprodukts, der typischerweise in erster Linie Frische und Sauberkeit vermitteln soll, mit komplexeren Parfümierungen, die erst zu einem späteren Zeitpunkt nach der Anwendung freigesetzt werden, zu kombinieren.

Aufgabe der vorliegenden Erfindung bestand daher darin, dem Verbraucher über den kompletten Anwendungszyklus eines Produktes ein verbessertes Dufterlebnis zu bieten. Eine solche Verbesserung der Geruchswahrnehmung ist durch den Wechsel von geruchlichen Profilen während der Anwendung eines Produktes und/oder der Anwendung einer mit dem Produkt ausgerüsteten Oberfläche, beispielsweise eines Textils, zu realisieren.

Es wurde nun überraschenderweise gefunden, dass das der vorliegenden Erfindung zugrundeliegende Problem gelöst werden kann durch den Einsatz von Kapseln, die weitere kleinere, duftstoffbeladene Kapseln ("Kapseln-in-Kapsel-Systeme") in Verbindung mit entsprechend konfektionierten Duftstoffzusammensetzungen enthalten.

In einem ersten Aspekt betrifft die Erfindung daher ein Mikrokapselsystem umfassend eine äußere Mikrokapsel mit einer äußeren Kapselhülle, wobei die äußere Mikrokapsel enthält:
(a) mindestens eine darin eingeschlossene innere Mikrokapsel mit einer inneren Kapselhülle; und
(b) eine erste Duftstoffzusammensetzung;
wobei die Kapselhülle der äußeren Mikrokapsel die innere Mikrokapsel und die erste Duftstoffzusammensetzung vollständig umgibt,
dadurch gekennzeichnet, dass
die innere Mikrokapsel eine zweite Duftstoffzusammensetzung enthält, welche vollständig von der inneren Kapselhülle der inneren Mikrokapsel umgeben ist und sich von der ersten Duftstoffzusammensetzung unterscheidet,
und dass die äußere Kapselhülle zumindest für Teile der ersten Duftstoffzusammensetzung partiell durchlässig ist und diese durch Diffusion freigesetzt werden.

In einem weiteren Aspekt betrifft die Erfindung Verfahren zur Herstellung der hierin beschriebenen Mikrokapselsysteme, welche umfassen (1) Bereitstellen von Mikrokapseln, die die zweite Duftstoffzusammensetzung enthalten, und einer ersten Duftstoffzusammensetzung, (2) Verkapseln der Mikrokapseln, die die zweite Duftstoffzusammensetzung enthalten, und der ersten Duftstoffzusammensetzung in einer äußeren Mikrokapsel.

In noch einem weiteren Aspekt betrifft die Erfindung Mittel zum Waschen, Reinigen, Konditionieren, Pflegen und/oder Färben von harten oder weichen Oberflächen, beispielsweise Textilien, Geschirr, etc., die das hierin beschriebene Mikrokapselsystem enthalten.

Des Weiteren wird ein Verfahren zur Erzeugung von polysensorischen Dufteindrücken unter Verwendung der hierin beschriebenen Mikrokapselsysteme beschrieben, wobei erst die Freisetzung der ersten Duftstoffzusammensetzung aus der äußeren Mikrokapsel und anschließend zeitlich verzögert die Freisetzung der zweiten Duftstoffzusammensetzung aus der inneren Mikrokapsel erfolgt. Bevorzugt erfolgt das Freisetzen der ersten Duftstoffzusammensetzung u.a. durch Diffusion durch die Kapselwand der äußeren Mikrokapsel sowie optional zusätzlich durch mechanische Beanspruchung. Bevorzugt erfolgt das Freisetzen der zweiten Duftstoffzusammensetzung durch mechanische Krafteinwirkung, insbesondere durch Reibung.

In noch einem weiteren Aspekt betrifft die Erfindung die Verwendung des beschriebenen Mikrokapselsystems zur Erzeugung polysensorischer Dufteindrücke.

Kapsel-in-Kapsel-Systeme sind beispielsweise allgemein aus der internationalen Patentveröffentlichung WO 02/060573 A2 bekannt. Dieses Dokument beschreibt die Verkapselung verschiedenster Aktivstoffe in Kapsel-in-Kapsel-Systemen, die auf mehr als eine Änderung der Umgebungseigenschaften reagieren können und eine gute bis erhöhte Schutzwirkung für die verkapselten Inhaltsstoffe bietet. Dabei sind die beschriebenen Systeme insbesondere für Anwendungen in Wasch- und Reinigungsmitteln, Kosmetika und Körperpflegemitteln und in der Klebstofftechnologie geeignet. Es werden allerdings keine Kapsel-in-Kapsel-Systeme zur Verkapselung von mindestens 2 verschiedenen Duftstoffzusammensetzungen beschrieben, die eine sequentielle Freisetzung zur Erzeugung von polysensorischen Dufteindrücken ermöglichen.

Diese und weitere Aspekte, Merkmale und Vorteile der Erfindung werden für den Fachmann aus dem Studium der folgenden detaillierten Beschreibung und Ansprüche ersichtlich. Dabei kann jedes Merkmal aus einem Aspekt der Erfindung in jedem anderen Aspekt der Erfindung eingesetzt werden. Ferner ist es selbstverständlich, dass die hierin enthaltenen Beispiele die Erfindung beschreiben und veranschaulichen sollen, diese aber nicht einschränken und insbesondere die Erfindung nicht auf diese Beispiele beschränkt ist.

Alle Prozentangaben sind, sofern nicht anders angegeben, Gewichts-% und beziehen sich jeweils auf die genannte Zusammensetzung. Numerische Bereiche, die in dem Format "von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

"Mindestens ein", wie hierin verwendet, bezieht sich auf 1 oder mehr, beispielsweise 1, 2, 3, 4, 5, 6, 7, 8, 9 oder mehr. Insbesondere bezieht sich diese Angabe auf die Art des Mittels/der Verbindung und nicht die absolute Zahl der Moleküle. "Mindestens ein Duftstoff", bedeutet daher, dass mindestens eine Art von Duftstoff erfasst wird, aber auch 2 oder mehr verschiedene Arten von Duftstoffen enthalten sein können.

"Etwa" oder "ungefähr", wie hierin im Zusammenhang mit einem Zahlenwert verwendet, bedeutet den Zahlenwert ±10%, vorzugsweise ±5%.

"Mikrokapselsystem", wie hierin verwendet, bezieht sich auf die hierin beschriebenen Kapsel-in-Kapsel-Systeme, d.h. Mikrokapseln, die wiederum Mikrokapseln einschließen.

"Mikrokapsel", wie hierin verwendet, bezieht sich auf Kapseln mit Kern-Schale Morphologie im Mikrometermaßstab, die eine Kapselhülle aufweisen, welche einen Kern vollständig einschließt. "Vollständig einschließt" oder "vollständig umgibt", wie hierin in Bezug auf die Mikrokapseln verwendet, bedeutet, dass der Kern vollständig von der Hülle umgeben ist, d.h. insbesondere nicht derart in eine Matrix eingebettet ist, dass er an einer Stelle freiliegt. Es ist ferner bevorzugt, dass die Kapselhülle derart beschaffen ist, dass die Freisetzung des Inhalts kontrolliert wird, d.h. der Inhalt nicht unabhängig von einem Freisetzungsreiz spontan unkontrolliert freigesetzt wird. Aus diesem Grund ist die Kapselhülle vorzugsweise im Wesentlichen undurchlässig für den verkapselten Inhalt. "Im Wesentlichen undurchlässig", wie in diesem Kontext verwendet, bedeutet, dass der Inhalt der Kapsel bzw. einzelne Inhaltsstoffe nicht spontan die Hülle durchdringen können, sondern die Freisetzung nur durch Öffnen der Kapsel oder optional auch über einen über einen längeren Zeitraum ablaufenden Diffusionsprozess erfolgen kann. Der Kern kann fest, flüssig und/oder gasförmig sein, ist aber vorzugsweise fest und/oder flüssig. Die Mikrokapseln sind vorzugsweise im Wesentlichen sphärisch und weisen Durchmesser im Bereich von 0,01 bis 1000 µm, insbesondere 0,1 bis 500 µm auf. Kapselhülle und Kapselkern bestehen aus unterschiedlichen Materialien, insbesondere ist die Kapselhülle bei Standardbedingungen (20°C, 1013 mbar) vorzugsweise fest, der Kern vorzugsweise fest und/oder flüssig, insbesondere flüssig.

Wenn im Folgenden allgemein auf "Mikrokapseln" Bezug genommen wird, ist selbstverständlich, dass die entsprechenden Angaben sowohl für die äußeren als auch die inneren Mikrokapseln gelten außer es ist explizit angegeben, dass die Angabe für eine der beiden Arten von eingesetzten Mikrokapseln zutrifft. Des Weiteren ist selbstverständlich, dass, auch wenn das erfindungsgemäße Kapselsystem hierin jeweils unter Bezugnahme auf eine äußere Mikrokapsel beschrieben wird, das eingesetzte Mikrokapselsystem üblicherweise eine Vielzahl solcher Mikrokapseln enthält, typischerweise >100, vorzugsweise >1000, oder daraus im Wesentlichen (d.h. zu 20 Gew.-% oder mehr, vorzugsweise 30 Gew.-% oder mehr, besonders bevorzugt 50 Gew.-% oder mehr) oder vollständig (d.h. zu 100 Gew.-%) besteht. Zusätzlich zu den Mikrokapseln kann das Mikrokapselsystem auch ein flüssiges Trägermedium, beispielsweise ein wässriges Trägermedium umfassen, in welchem die äußeren Mikrokapseln dispergiert sind, um so beispielsweise ein Kapselslurry zu bilden. In solchen Kapselslurrys machen die Mikrokapseln der Erfindung typischerweise 10 bis 80 Gew.-%, vorzugsweise 20 bis 50 Gew.-% aus.

Als Kapselmaterial für die erfindungsgemäß Mikrokapseln können ganz allgemein z. B. hochmolekulare Verbindungen tierischer oder pflanzlicher Herkunft, z. B. Eiweißverbindungen (Gelatine, Albumin, Casein), Cellulose-Derivate (Methylcellulose, Ethylcellulose, Celluloseacetat, Cellulosenitrat, Carboxymethylcellulose) sowie insbesondere synthetische Polymere (z. B. Polyamide, Polyolefine, Polyester, Polyurethane, Epoxidharze, Silikonharze und Kondensationsprodukte von Carbonyl- und NH-Gruppen-haltigen Verbindungen) verwendet werden. Konkret kann das Schalenmaterial beispielsweise ausgewählt werden aus Polyacrylaten; Polyethylen; Polyamiden; Polystyrolen; Polyisoprenen; Polycarbonaten; Polyestern; Polyharnstoffen; Polyurethanen; Polyolefinen; Polysacchariden; Epoxidharzen; Vinylpolymeren; Harnstoff vernetzt mit Formaldehyd oder Glutaraldehyd; Melamin vernetzt mit Formaldehyd; Gelatine-Polyphosphat-Koazervaten, optional vernetzt mit Glutaraldehyd; Gelatine-Gummi Arabicum Koazervaten; Silikonharze; mit Polyisocyanaten umgesetzten Polyaminen; mittels freier Radikalpolymerisation polymerisierter Acrylatmonomere; Seide; Wolle; Gelatine; Cellulose; Proteinen; und Mischungen und Copolymeren der vorgenannten. Besonders bevorzugt sind Polyacrylate, Polyethylen, Polyamide, Polystyrole, Polyisoprene, Polycarbonate, Polyester, Polyharnstoffe, Polyurethane, Polyolefine, Epoxidharze, Vinylpolymere und Harnstoff und/oder Melamin vernetzt mit Formaldehyd oder Glutaraldehyd.

Zur Herstellung der erfindungsgemäßen Mikrokapseln sind prinzipiell die bekannten Mikroverkapselungsverfahren geeignet, bei denen z. B. die Einkapselung der einzukapselnden Phase durch Umhüllung mit filmbildenden Polymeren (wie z. B. zuvor genannt), die sich nach Emulgierung und Koazervation oder Grenzflächenpolymerisation auf dem einzuhüllenden Material niederschlagen, erfolgt. In Bezug auf die vorliegende Erfindung handelt es sich bei der einzukapselnden Phase vorzugsweise um eine Duftstoffzusammensetzung, üblicherweise in Form eines Parfümöls. Die inneren Mikrokapseln werden vorzugsweise mittels den vorstehend genannten, bekannten Verfahren hergestellt, indem die zweite Duftstoffzusammensetzung, die üblicherweise in Form eines Parfümöls vorliegt, in geeigneten filmbildenden Polymeren verkapselt wird. Zur Herstellung der äußeren Mikrokapseln werden die inneren Mikrokapseln vorzugsweise in der ersten Duftstoffzusammensetzung, üblicherweise wiederum ein Parfümöl, welches sich von dem in den inneren Kapseln unterscheidet, dispergiert und diese Dispersion dann in filmbildenden Polymeren verkapselt.

Die Mikrokapseln können die enthaltenen Duftstoffzusammensetzungen durch verschiedene Einwirkungen aus der Umwelt freisetzen, vorzugsweise bei Änderung des pH-Wertes oder der lonenstärke der Umgebung, bei Änderung der Temperatur, bei Einwirkung von Licht, durch Diffusion und/oder bei mechanischer Beanspruchung. Es kann bevorzugt sein, dass sich die äußere Mikrokapsel, d.h. die Kapselhülle der äußeren Mikrokapsel, und die innere Mikrokapsel, d.h. die Kapselhülle der inneren Mikrokapsel, in ihrem Aufbau oder der Zusammensetzung unterscheiden, so dass unterschiedliche Freisetzungsmechanismen zum Einsatz kommen bzw., wenn derselbe Freisetzungsmechanismus verwendet wird, unterschiedliche Freisetzungsbedingungen eingesetzt werden. "Unterschiedliche Freisetzungsbedingungen", wie hierin verwendet, bezieht sich auch auf unterschiedliche Durchlässigkeiten der Kapselhüllen, wie bereits oben definiert. In verschiedenen Ausführungsformen ist es bevorzugt, dass sich die äußeren und inneren Mikrokapseln in ihrem Freisetzungsverhalten, d.h. der Freisetzung des verkapselten Materials, beispielsweise der Duftstoffzusammensetzungen, unterscheiden. Ein derartiger Unterschied im Freisetzungsverhalten bezieht sich auf die Kapseln als solche im direkten Vergleich, d.h. in einem derartigen Vergleich werden die inneren Kapseln in freier, unverkapselter Form mit den äußeren Kapseln als solche, d.h. ohne darin verkapselte innere Kapseln, im Hinblick auf das Freisetzungsverhalten verglichen. In verschiedenen Ausführungsformen unterscheiden sich innere und äußere Kapseln bei Untersuchung eines gegebenen Freisetzungsmechanismus, beispielsweise der Diffusivität, in ihrem Freisetzungsverhalten. Eine solche Untersuchung kann beispielsweise mittels thermogravimetrischer Analyse (TGA) mit einer Heizrate von 1K/min gekoppelt mit Fast Fourier Infrarotspektroskopie (FFIR) im einem Temperaturbereich von Raumtemperatur (20°C) bis 350°C unter Stickstoffatmosphäre (beispielsweise 1,8 I N₂/h) erfolgen. Dabei erfolgt eine Einwaage der Kapseln im Mengenbereich von 10-12 mg und Untersuchung in AI-Tiegeln. Beide Arten von Kapseln sind zum Zwecke dieser Vergleichstest mit derselben Duftstoffmischung gefüllt. Vor jeder Messung wird eine Hintergrundmessung durchgeführt, wobei die Signale des Messspektrums dann immer sofort um den Hintergrund korrigiert werden. Wenn hierin darauf Bezug genommen wird, dass sich innere und äußere Kapseln in ihrem Freisetzungsverhalten unterscheiden, bedeutet dies - sofern nicht anders angegeben - dass sich die Kapseln derart in ihrer Durchlässigkeit für die verkapselten Substanzen unterscheiden, dass während der dynamischen Phase (d.h. während dem Heizen) der TGA-FFIR unter den oben angegebenen Bedingungen zu irgendeinem Zeitpunkt, beispielsweise im Temperaturbereich zwischen 80 und 300°C, bei gleicher Temperatur ein Unterschied im Gewichtsverlust relativ zum Anfangsgewicht von mindestens 1% ergibt. In verschiedenen Ausführungsformen ist der Gewichtsverlust der äußeren Kapseln unter gleichen Bedingungen bei derselben Temperatur (beispielsweise nach 250 Minuten Heizen mit 1K/min und einer Temperatur von 280°) mindestens 1 Gew. % größer als der der inneren Kapseln. Das bedeutet beispielsweise, dass nach einer Zeit von 250 Minuten und bei einer Temperatur von 280°C der Gewichtsverlust (relativ zum Ausgangsgewicht) der äußeren Kapsel 86,6% beträgt, während der der inneren Kapsel 82,3% beträgt (d.h. ein Unterschied von 4,3%).

Erfindungsgemäß ist die äußere Kapselhülle zumindest für Teile der ersten Duftstoffzusammensetzung partiell durchlässig und setzt diese durch Diffusion frei. So kann die Freisetzung der ersten Duftstoffzusammensetzung durch Diffusion und optional zusätzlich mechanische Freisetzung, und die Freisetzung der zweiten Duftstoffzusammensetzung durch einen davon unterschiedlichen Freisetzungsmechanismus erfolgen, insbesondere nur durch mechanische Beanspruchung. Dazu ist es erforderlich, dass die Kapselmaterialien für äußere und innere Kapselhülle entsprechend unterschiedlich beschaffen sind. Die äußeren und inneren Mikrokapseln unterscheiden sich daher durch ihr Freisetzungsverhalten. Generell ist es bevorzugt, dass die äußeren Mikrokapseln die erste Duftstoffzusammensetzung langsam freisetzen, erfindungsgemäß insbesondere dadurch, dass die Kapselhülle diffusiv durchlässig ist, wohingegen die inneren Mikrokapseln zurückgehalten werden und erst auf einen späteren Reiz hin aufgebrochen werden. Die Freisetzung der inneren Mikrokapseln erfolgt üblicherweise, selbst wenn die äußeren Mikrokapseln diffusiv durchlässig sind, nicht durch Diffusion, sondern dadurch dass die äußere Mikrokapsel durch einen der übrigen oben genannten Freisetzungsmechanismen aufgebrochen wird. Dabei kann der Unterschied in der Diffusivität auch dadurch bewirkt werden, dass die inneren Kapseln selber verkapselt sind und daher die Diffusivität selbst bei gleichem Aufbau der Hülle durch die Verkapselung in den äußeren Kapseln eingeschränkt wird. In verschiedenen Ausführungen wird dieser Unterschied aber ferner dadurch verstärkt, dass die Kapselhülle der inneren Kapseln anders beschaffen ist als die der äußeren Kapseln, d.h. ein wie oben beschriebener Unterschied im Freisetzungsverhalten besteht. Wenn also hierin vom Freisetzungsmechanismus mittels Diffusion die Rede ist, bezieht sich diese Angabe immer auf die Duftstoffe und nicht die inneren Mikrokapseln. Mit "diffusiv durchlässig" bezogen auf die äußeren Kapseln ist hierin gemeint, dass die Durchlässigkeit gegenüber den Duftstoffmolekülen mittels Diffusion größer ist als die entsprechende Durchlässigkeit der inneren Kapseln. Der Ausdruck wird hierin daher im Wesentlichen als relativer Begriff verwendet.

Die Diffusivität (Durchlässigkeit gegenüber Diffusion) der Kapseln kann beispielsweise über den Vernetzungsgrad der Hüllmaterialien und die Wandstärke der Kapseln eingestellt werden.

Bei den erfindungsgemäßen Mikrokapseln kann es sich vorzugsweise um wasserlösliche und/oder wasserunlösliche Mikrokapseln handeln.

Bevorzugt handelt es sich insbesondere bei den äußeren Mikrokapseln aber um wasserunlösliche Mikrokapseln. Die Wasserunlöslichkeit der äußeren Mikrokapseln hat den Vorteil, dass hierdurch bei Einsatz entsprechender Wasch- oder Reinigungsmittel eine die Anwendung überdauernde Trennung von Duftstoffen ermöglicht werden kann, sowie dass die Duftstofffreisetzung aus den Mikrokapseln auch nach der Anwendung erfolgen/andauern kann.

Auch die inneren Mikrokapseln sind aus den oben genannten Gründen vorzugsweise wasserunlöslich.

Das Wandmaterial der Mikrokapseln umfasst vorzugsweise Polyurethane, Polyolefine, Polyamide, Polyester, Polysaccharide, Epoxidharze, Silikonharze und/oder Polykondensationsprodukte aus Carbonyl-Verbindungen und NH-Gruppen enthaltenden Verbindungen. Dies entspricht einer bevorzugten Ausführungsform der Erfindung. Bevorzugt können beispielsweise Melamin-Harnstoff-Formaldehyd-Mikrokapseln oder Melamin-Formaldehyd-Mikrokapseln oder Harnstoff-Formaldehyd-Mikrokapseln eingesetzt werden. Besonders bevorzugt sind Mikrokapseln auf Basis von Melamin-Formaldehyd-Harzen. In bevorzugten Ausführungsformen sind somit die äußeren Mikrokapseln und die inneren Mikrokapseln solche auf Basis von Melamin-Formaldehyd-Harzen.

Das allgemeine Vorgehen bei der Mikrokapselherstellung als solches ist dem Fachmann seit langem wohlbekannt. Besonders geeignete Verfahren zur Mikrokapselherstellung sind prinzipiell z. B. in US 3,516,941, in US 3,415,758 oder auch in EP 0 026 914 A1 beschrieben. Letztgenannte beschreibt beispielsweise die Mikrokapselherstellung durch säureinduzierte Kondensation von Melamin-Formaldehyd-Vorkondensaten und/oder deren C1-C4-Alkylethern in Wasser, in dem das den Kapselkern bildende hydrophobe Material dispergiert ist, in Gegenwart eines Schutzkolloids.

In besonders bevorzugten Ausführungsformen sind die äußeren Mikrokapseln solche auf Basis von Melamin-Formaldehyd-Harzen und die Freisetzung der verkapselten Duftstoffe erfolgt zumindest teilweise auf diffusivem Weg. In solchen Ausführungsformen ist die Kapselhülle für die verkapselte Duftstoffzusammensetzung oder Bestandteile davon derart durchlässig, dass eine, vorzugsweise anhaltende, Diffusion der Riechstoffmoleküle in die Umgebungsluft auftritt. Zusätzlich ist die äußere Mikrokapsel vorzugsweise durch mechanische Beanspruchung aufbrechbar, insbesondere aufreibbar (engl. "friable"). Letzterer Mechanismus führt zum Aufbrechen der äußeren Kapselschale und damit zur Freisetzung der inneren Mikrokapseln.

In weiter bevorzugten Ausführungsformen sind die inneren Mikrokapseln solche auf Basis von Melamin-Formaldehyd-Harzen und die Freisetzung erfolgt durch mechanische Beanspruchung, insbesondere sind die inneren Mikrokapseln aufreibbar (engl. "friable"). Die inneren Mikrokapseln sind in solchen Ausführungsformen für die verkapselte Duftstoffzusammensetzung vorzugsweise im Wesentlichen undurchlässig, d.h. die Diffusion der verkapselten Duftstoffmoleküle ist derart eingeschränkt, dass keine oder nur ein sehr geringer Teil, der üblicherweise unter der Wahrnehmungsgrenze liegt, der Riechstoffmoleküle die Kapselhülle mittels Diffusion durchdringt.

In bevorzugten Ausführungsformen ist die Kapselhülle der äußeren Mikrokapsel für die verkapselte erste Duftstoffzusammensetzung durchlässiger als die Kapselhülle der inneren Mikrokapsel für die verkapselte zweite Duftstoffzusammensetzung. "Durchlässiger" bedeutet in diesem Zusammenhang, dass die absolute Menge der Duftstoffmoleküle, die durch die geschlossene Schale über einen festgelegten Zeitraum hindurch diffundiert größer ist als die Referenz. Die Menge ist beispielsweise mindestens um den Faktor 2, mindestens um den Faktor 10, oder mindestens um den Faktor 100 oder 1000 größer. Wenn hierin von Durchlässigkeit oder Diffusion gesprochen wird, beziehen sich diese Angaben immer auf die Kapseln nach der Anwendung, d.h. beispielsweise nachdem diese auf eine Oberfläche, wie z.B. ein Textil, aufgezogen sind. Die Diffusion erfolgt damit vorzugsweise in die Umgebungsluft, vorzugsweise aus einer Kapsel, die bereits weitgehend von den übrigen Bestandteilen des Mittels, in welchem sie formuliert war, getrennt ist.

In solchen bevorzugten Ausführungsformen werden sowohl die äußeren als auch die inneren Mikrokapseln durch mechanische Beanspruchung aufgebrochen und der Inhalt freigesetzt, die Kapseln unterscheiden sich aber dadurch, dass die äußeren Mikrokapseln für die darin verkapselten Duftstoffe teilweise durchlässig, d.h. zumindest durchlässiger als die inneren Mikrokapseln, sind, so dass diese durch Diffusion nach und nach freigesetzt werden können, während die inneren Mikrokapseln weitgehend undurchlässig, d.h. weniger durchlässig als die äußeren Mikrokapseln, für die darin verkapselten Duftstoffe sind, so dass deren Freisetzung erst nach dem Aufbrechen der inneren Kapselhülle erfolgt. Die äußeren Mikrokapseln erlauben aber vorzugsweise nicht die Freisetzung der inneren Mikrokapseln, bevor die äußere Hülle durch mechanische Beanspruchung aufgebrochen wird. Da die äußeren Mikrokapseln durch mechanische Beanspruchung aufgebrochen werden, werden die inneren Mikrokapseln freigesetzt, die wiederum ebenfalls durch mechanische Beanspruchung aufgebrochen werden. So kommt es zur Freisetzung der nicht bereits durch Diffusion freigesetzten ersten Duftstoffzusammensetzung und gleichzeitig zur Freisetzung der zweiten Duftstoffzusammensetzung. Die Unterschiede in der Durchlässigkeit zwischen äußeren und inneren Mikrokapseln sind dabei beispielsweise wie oben definiert und mittels TGA-FFIR messbar.

Der Begriff "aufreibbare Mikrokapseln" meint solche Mikrokapseln, welche durch mechanisches Reiben oder durch Druck, wie er z. B beim Abtrocknen der Hände mit einem Handtuch entsteht, geöffnet bzw. aufgerieben werden können, so dass eine Inhaltsfreisetzung im Wesentlichen erst als Resultat einer mechanischen Einwirkung resultiert, beispielsweise wenn man sich mit einem Handtuch, auf welchem solche Mikrokapseln abgelagert sind, die Hände abtrocknet. Die bevorzugt verwendeten Melamin-Formaldehyd-Harz-basierten Mikrokapseln sind typischerweise derartige aufreibbare Mikrokapseln.

In verschiedenen Ausführungsformen weisen Kapseln für welche eine Durchlässigkeit per Diffusion weitgehend unerwünscht ist, wie beispielsweise die inneren Kapseln, >10 bis 20% Wandmaterial, vorzugsweise 12 bis 18, noch bevorzugter 13-17, am bevorzugtesten 14-16% Wandmaterial (=Hülloder Schalenmaterial) bezogen auf das Gesamtgewicht der Kapsel auf, wobei der Rest durch das Kernmaterial gebildet wird. In Verbindung mit einem üblichen, geeigneten Vernetzungsgrad führt dies beispielsweise zu einer ausreichenden Diffusionsbarriere der inneren Kapsel. Die inneren Kapseln weisen daher in verschiedenen Ausführungsformen einen Anteil von >10 bis 20% Wandmaterial, vorzugsweise 12 bis 18, noch bevorzugter 13-17, am bevorzugtesten 14-16% Wandmaterial relativ zum Gesamtgewicht der Kapseln auf.

In verschiedenen weiteren Ausführungsformen führt eine Reduktion des Wandmaterials der Kapsel von >10% zu einer erhöhten, schnelleren und wahrnehmbaren Diffusion des Kernmaterials. Dementsprechend zeichnen sich die äußeren Kapseln in verschiedenen Ausführungsformen dadurch aus, dass der Anteil des Wandmaterials am Gesamtgewicht der Kapseln gegenüber den inneren Kapseln, bei gleichem Material und Vernetzungsgrad, um mehr als 10%, beispielweise >10 bis 30%, verringert ist. Beispielsweise kann die innere Kapsel einen Anteil an Wandmaterial von 14-16% aufweisen, wobei dann die äußere Kapsel einen Anteil an Wandmaterial von 13 % oder weniger, beispielsweise 10-12%, aufweist.

Das Freisetzungsverhalten kann ferner auch, abhängig von dem Wandmaterial (die Ausdrücke "Wandmaterial", "Hüllmaterial" und "Schalenmaterial" werden hierin austauschbar verwendet) durch den Vernetzungsgrad der Kapseln reguliert werden. Dieser wird neben den Reaktionsbedingungen (z.B. pH-Wert, Zeit, Temperatur) auch - bei Melamin-Formaldehyd-Kapseln - durch das molare Verhältnis von Formaldehyd zu Melamin bestimmt.

Hier hat es sich in verschiedenen Ausführungsformen erwiesen, dass das molare Verhältnis von Formaldehyd zu Melamin für die innere Kapsel auf > 1:1, beispielsweise 1,05:1 bis 1,25:1, und für die äußere Kapsel auf < 1:1, beispielsweise 0,95:1 bis 0,75:1, eingestellt werden kann, um die gewünschten Unterschiede im Freisetzungsverhalten zu erzielen. Diese Unterschiede können wie oben beschrieben mittels TGA-FFIR quantifiziert werden.

In verschiedenen Ausführungsformen werden diese Merkmale hinsichtlich der Menge des Wandmaterials und des Vernetzungsgrades, beispielsweise mittels des Verhältnisses Formaldehyd-Melamin, kombiniert, um die gewünschten Unterschiede im Freisetzungsverhalten zu erzielen.

Es hat sich beim Einsatz der erfindungsgemäßen Kapselsysteme überraschend gezeigt, dass der Duftwechsel zu einem synergistischen Effekt führt und die Duftintensität höher bewertet wird. Des Weiteren ermöglichen diese Kapsel-in-Kapsel-Systeme eine schärfere Trennung der verschiedenen Düfte, da die Freisetzung der Duftstoffe aus der inneren Kapsel, insbesondere wenn diese ohnehin schon im Wesentlichen undurchlässig für die Duftstoffe ist, durch die Verkapselung in einer äußeren Mikrokapsel weiter erschwert wird. Dadurch unterscheidet sich das Geruchsprofil derartiger Kapsel-in-Kapsel-Systemen von Systemen in denen zwei verschiedene Mikrokapseln mit jeweils der ersten und der zweiten Duftstoffzusammensetzungen entsprechenden Parfümierungen und Kapselmorphologien (d.h. eine Kapsel ist diffusiv durchlässig während die andere im Wesentlichen undurchlässig ist), da der Einsatz von zwei separaten Kapseln immer zum einem gewissen Grad zum Vermischen der einzelnen Duftstoffzusammensetzungen führt, wohingegen dieses Vermischen der Geruchseindrücke durch die beanspruchten Kapsel-in-Kapsel-Systemen signifikant verringert wird, so dass die verschiedenen Gerüche als schärfer getrennt wahrgenommen werden können.

Bevorzugte erfindungsgemäße Mikrokapseln weisen mittlere Durchmesser (Median der Größenverteilung) im Bereich von 0,1 bis 500 µm auf, vorzugsweise zwischen 1 und 150 µm, insbesondere zwischen 1 und 100 µm, z. B. 10 bis 80 µm. Die den Kern bzw. (gefüllten) Hohlraum umschließende Schale der Mikrokapseln hat eine durchschnittliche Dicke im Bereich zwischen vorteilhafterweise rund 1 nm und 1000 nm, vorzugsweise zwischen rund 10 nm und etwa 500 nm, bevorzugter zwischen rund 30 nm und etwa 300 nm, noch bevorzugter 30 nm bis 200 nm, insbesondere etwa 50 nm bis ungefähr 150 nm.

Dabei weisen die erfindungsgemäßen äußeren Mikrokapseln vorzugsweise mittlere Durchmesser (Median der Größenverteilung) von 5 bis 500 µm, vorzugsweise 10 bis 150 µm, noch bevorzugter 15 bis 100 µm bei Schalendicken von 30 nm bis 200 nm auf und die inneren Mikrokapseln mittlere Durchmesser von 1 bis 30 µm, vorzugsweise 2 bis 25 µm, noch bevorzugter 5 bis 20 µm, bei Schalendicken von 30 nm bis 200 nm. Über die Schalendicke kann unter anderem auch die Durchlässigkeit der Schale, d.h. die Diffusivität, kontrolliert werden. Niedrige Schalendicken bedingen dabei eine höhere diffusive Durchlässigkeit als größere Schalendicken. In verschiedenen Ausführungsformen ist die Schalendicke der äußeren Mikrokapsel in etwa gleich groß oder kleiner als die der inneren Mikrokapsel.

In bevorzugten Ausführungsformen der Erfindung enthält die äußere Mikrokapsel durchschnittlich mehr als eine, vorzugsweise mindestens 2, noch bevorzugter mindestens 3, am bevorzugtesten 4 oder mehr darin eingeschlossene innere Mikrokapseln. In verschiedenen Ausführungsformen kann die äußere Mikrokapsel bis zu 20, vorzugsweise bis zu 15, noch bevorzugter bis zu 10 innere Mikrokapseln enthalten.

In verschiedenen Ausführungsformen kann der mittlere Durchmesser der inneren Kapseln mindestens um den Faktor 2 kleiner als der mittlere Durchmesser der äußeren Kapseln sein, vorzugsweise ist dieser um den Faktor 2-10 kleiner, insbesondere 2-5. Die äußeren Mikrokapseln haben beispielsweise einen mittleren Durchmesser von 20 bis 80 µm, die inneren von 1 bis 20 µm.

Die inneren Mikrokapseln sind vorzugsweise in der ebenfalls in den äußeren Mikrokapseln verkapselten ersten Duftstoffzusammensetzung dispergiert. Sowohl die Kapselhülle der äußeren als auch der inneren Mikrokapseln ist daher in der ersten Duftstoffzusammensetzung unlöslich.

Die erste und zweite Duftstoffzusammensetzung enthalten jeweils mindestens einen Duftstoff. Als Duftstoffe bzw. Riechstoffe bzw. Parfümöle können alle dafür bekannten Stoffe und Gemische eingesetzt werden. Im Sinne dieser Erfindung werden die Begriffe "Riechstoff(e)", "Duftstoffe" und "Parfümöl(e)" synonym gebraucht. Damit sind insbesondere all jene Stoffe oder deren Gemische gemeint, die von Mensch und Tier als Geruch empfunden werden, insbesondere vom Mensch als Wohlgeruch empfunden werden.

Als Duftkomponenten können Parfüme, Parfümöle oder Parfümölbestandteile eingesetzt werden. Parfümöle bzw. Duftstoffe können erfindungsgemäß einzelne Riechstoffverbindungen, z. B. die synthetischen Produkte vom Typ der Ester, Ether, Aldehyde, Ketone, Alkohole und Kohlenwasserstoffe sein.

Duftstoffverbindungen vom Typ der Aldehyde sind beispielsweise Adoxal (2,6,10-Trimethyl-9-undecenal), Anisaldehyd (4-Methoxybenzaldehyd), Cymal (3-(4-Isopropyl-phenyl)-2-methylpropanal), Ethylvanillin, Florhydral (3-(3-isopropylphenyl)butanal), Helional (3-(3,4-Methylendioxyphenyl)-2-methylpropanal), Heliotropin, Hydroxycitronellal, Lauraldehyd, Lyral (3- und 4-(4-Hydroxy-4-methylpentyl)-3- cyclohexen-1-carboxaldehyd), Methylnonylacetaldehyd, Lilial (3-(4-tert-Butylphenyl)-2-methylpropanal), Phenylacetaldehyd, Undecylenaldehyd, Vanillin, 2,6,10-Trimethyl-9-undecenal, 3-Dodecen-1-al, alpha-n-Amylzimtaldehyd, Melonal (2,6-Dimethyl-5-heptenal), 2,4-Di-methyl-3-cyclohexen-1-carboxaldehyd (Triplal), 4-Methoxybenzaldehyd, Benzaldehyd, 3-(4-tert- Butylphenyl)-propanal, 2-Methyl-3-(para-methoxyphenyl)propanal, 2-Methyl-4-(2,6,6-timethyl-2(1)-cyclohexen-1-yl)butanal, 3-Phenyl-2-propenal, cis-/trans-3,7-Dimethyl-2,6-octadien-1-al, 3,7-Dimethyt-6-octen-1-al, [(3,7-Dimethyl-6-octenyl)oxy]acetaldehyd, 4-Isopropylbenzylaldehyd, 1,2,3,4,5,6,7,8-Octahydro-8,8-dimethyl-2-naphthaldehyd, 2,4-Dimethyl-3-cyclohexen-1-carboxaldehyd, 2-Methyl-3-(isopropylphenyl)propanal, 1-Decanal, 2,6-Dimethyl-5-heptenal, 4-(Tricyclo[5.2.1.0(2,6)]-decyliden-8)-butanal, Octahydro-4,7-methan-1H-indencarboxaldehyd, 3-Ethoxy-4-hydroxybenzaldehyd, para-Ethyl-alpha,alpha-dimethylhydrozimtaldehyd, alpha-Methyl-3,4-(methylendioxy)-hydrozimtaldehyd, 3,4-Methylendioxybenzaldehyd, alpha-n-Hexylzimtaldehyd, m-Cymen-7-carboxaldehyd, alpha-Methylphenylacetaldehyd, 7-Hydroxy-3,7-dimethyloctanal, Undecenal, 2,4,6-Trimethyl-3-cyclohexen-1-carboxaldehyd, 4-(3)(4-Methyl-3-pentenyl)-3-cyclohexencarboxaldehyd, 1-Dodecanal, 2,4-Dimethylcyclohexen-3-carboxaldehyd, 4-(4-Hydroxy-4-methylpentyl)-3-cylohexen-1-carboxaldehyd, 7-Methoxy-3,7-dimethyloctan-1-al, 2-Methyl- undecanal, 2-Methyldecanal, 1-Nonanal, 1-Octanal, 2,6,10-Trimethyl-5,9-undecadienal, 2-Methyl-3-(4-tert-butyl)propanal, Dihydrozimtaldehyd, 1-Methyl-4-(4-methyl-3-pentenyl)-3-cyclohexen-1-carboxaldehyd, 5- oder 6-Methoxyhexahydro-4,7-methanindan-1-oder -2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexencarboxaldehyd, 7-Hydroxy-3J-dimethyl-octanal, trans-4-Decenal, 2,6-Nonadienal, para-Tolylacetaldehyd, 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexen- carboxaldehyd, 3J-Dimethyl-2-methylen-6-octenal, Phenoxyacetaldehyd, 5,9-Dimethyl-4,8- decadienal, Päonienaldehyd (6,10-Dimethyl-3-oxa-5,9-undecadien-1-al), Hexahydro-4,7-methanindan-1-carboxaldehyd, 2-Methyloctanal, alpha-Methyl-4-(1-methylethyl)benzolacetaldehyd, 6,6-Dimethyl-2-norpinen-2-propionaldehyd, para-Methylphenoxyacetaldehyd, 2-Methyl-3-phenyl-2-propen-1-al, 3,5,5-Trimethylhexanal, Hexahydro-8,8-dimethyl-2-naphthaldehyd, 3-Propyl-bicyclo-[2.2.1]-hept-5-en-2-carbaldehyd, 9-Decenal, 3-Methyl-5-phenyl-1-pentanal, Methylnonylacetaldehyd, Hexanal und trans-2-Hexenal.

Duftstoffverbindungen vom Typ der Ketone sind beispielsweise Methyl-beta-naphthylketon, Moschusindanon (1,2,3,5,6,7-Hexahydro-1,1,2,3,3- pentamethyl-4H-inden-4-on), Tonalid (6-Acetyl-1,1,2,4,4,7-hexamethyltetralin), alpha-Damascon, beta-Damascon, delta-Damascon, iso-Damascon, Damascenon, Methyldihydrojasmonat, Menthon, Carvon, Kampfer, Koavon (3,4,5,6,6-Pentamethylhept-3-en-2-on), Fenchon, alpha-lonon, beta- Ionon, gamma-Methyl-lonon, Fleuramon (2-heptylcyclopen-tanon), Dihydrojasmon, cis-Jasmon, iso-E-Super (1-(1,2,3,4,5,6J,8-octahydro-2,3,8,8-tetramethyl-2-naphthalenyl)-ethan-1-on (und Isomere)), Methylcedrenylketon, Acetophenon, Methylacetophenon, para-Methoxyacetophenon, Methyl-beta-naphtylketon, Benzylaceton, Benzophenon, para-Hydroxyphenylbutanon, Sellerie- Keton(3-methyl-5-propyl-2-cyclohexenon), 6-Isopropyldecahydro-2-naphton, Dimethyloctenon, Frescomenthe (2-butan-2-yl-cyclohexan-1-on), 4-(1-Ethoxyvinyl)-3,3,5,5-tetramethylcyclohexanon, Methylheptenon, 2-(2-(4-Methyl-3-cyclohexen-1-yl)propyl)cyclopentanon, 1-(p-Menthen-6(2)yl)-1-propanon, 4-(4-Hydroxy-3-methoxyphenyl)-2-butanon, 2-Acetyl-3,3-dimethylnorbornan, 6,7- Dihydro-1,1,2,3,3-pentamethyl-4(5H)-indanon, 4-Damascol, Dulcinyl(4-(1,3-benzodioxol-5-yl) butan-2-on), Hexalon (1-(2,6,6-trimethyl-2-cyclohexene-1-yl)-1,6-heptadien-3-on), IsocyclemonE(2-acetonaphthon-1,2,3,4,5,6,7,8-octahydro-2,3,8,8-tetramethyl), Methylnonylketon, Methylcyclocitron, Methyllavendelketon, Orivon (4-tert-Amyl-cyclohexanon), 4-tert-Butylcyclohexanon, Delphon (2-pentyl-cyclopentanon), Muscon (CAS 541-91-3), Neobutenon (1-(5,5-dimethyl-1- cyclohexenyl)pent-4-en-1-on), Plicaton (CAS 41724-19-0), Velouton (2,2,5-Trimethyl-5-pentylcyclopentan-1-on),2,4,4,7-Tetramethyl-oct-6-en-3-on und Tetrameran (6,10- Dimethylundecen-2-on).

Duftstoffverbindungen vom Typ der Alkohole sind beispielsweise 10-Undecen-1-ol, 2,6-Dimethylheptan-2-ol, 2-Methyl-butanol, 2-Methylpentanol, 2- Phenoxyethanol, 2-Phenylpropanol, 2-tert.-Butycyclohexanol, 3,5,5-Trimethylcyclohexanol, 3-Hexanol, 3-Methyl-5-phenyl-pentanol, 3-Octanol, 3-Phenyl-propanol, 4-Heptenol, 4-Isopropyl- cyclohexanol, 4-tert.-Butycyclohexanol, 6,8-Dimethyl-2-nona-nol, 6-Nonen-1-ol, 9-Decen-1-ol, α-Methylbenzylalkohol, α-Terpineol, Amylsalicylat, Benzylalkohol, Benzylsalicylat, β-Terpineol, Butylsalicylat, Citronellol, Cyclohexylsalicylat, Decanol, Dihydromyrcenol, Dimethylbenzylcarbinol, Dimethylheptanol, Dimethyloctanol, Ethylsalicylat, Ethylvanilin, Eugenol, Farnesol, Geraniol, Heptanol, Hexylsalicylat, Isoborneol, Isoeugenol, Isopulegol, Linalool, Menthol, Myrtenol, n-Hexanol, Nerol, Nonanol, Octanol, p-Menthan-7-ol, Phenylethylalkohol, Phenol, Phenylsalicylat, Tetrahydrogeraniol, Tetrahydrolinalool, Thymol, trans-2-cis-6-Nonadicnol, trans-2-Nonen-1-ol, trans-2-Octenol, Undecanol, Vanillin, Champiniol, Hexenol und Zimtalkohol.

Duftstoffverbindungen vom Typ der Ester sind z.B. Benzylacetat, Phenoxyethylisobutyrat, p-tert-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmacyclat.

Zu den Ethern zählen beispielsweise Benzylethylether und Ambroxan. Zu den Kohlenwasserstoffen gehören hauptsächlich Terpene wie Limonen und Pinen.

Bevorzugt werden Mischungen verschiedener Duftstoffe verwendet, die gemeinsam eine ansprechende Duftnote erzeugen. Ein derartiges Gemisch an Duftstoffen kann auch als Parfüm oder Parfümöl bezeichnet werden. Solche Parfümöle können auch natürliche Duftstoffgemische enthalten, wie sie aus pflanzlichen Quellen zugänglich sind.

Zu den Duftstoffen pflanzlichen Ursprungs zählen ätherische Öle wie Angelikawurzelöl, Anisöl, Arnikablütenöl, Basilikumöl, Bayöl, Champacablütenöl, Citrusöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, jasminöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Labdanumöl, Lavendelöl, Lemongrasöl, Lindenblütenöl, Limettenöl, Mandarinenöl, Melissenöl, Minzöl, Moschuskörneröl, Muskatelleröl, Myrrhenöl, Nelkenöl, Neroliöl, Niaouliöl, Olibanumöl, Orangenblütenöl, Orangenschalenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Salbeiöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl sowie Ambrettolid, Ambroxan, alpha-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, Boisambrene forte, alpha-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-beta-naphthylketon, Methyl-n-nonylacetaldehyd, Methyl-n-nonylketon, Muskon, beta-Naphtholethylether, beta-Naphthol-methylether, Nerol, n-Nonylaldehyd, Nonylalkohol, n-Octylaldehyd, p-Oxy-Acetophenon, Pentadekanolid, beta-Phenylethylalkohol, Phenylessigsäure, Pulegon, Safrol, Salicylsäureisoamylester, Salicylsäuremethylester, Salicylsäurehexylester, Salicylsäurecyclohexylester, Santalol, Sandelice, Skatol, Terpineol, Thymen, Thymol, Troenan, gamma-Undelacton, Vanillin, Veratrumaldehyd, Zimtaldehyd, Zimtalkohol, Zimtsäure, Zimtsäureethylester, Zimtsäurebenzylester, Diphenyloxid, Limonen, Linalool, Linalylacetat und - Propionat, Melusat, Menthol, Menthon, Methyl-n-heptenon, Pinen, Phenylacetaldehyd, Terpinylacetat, Citral, Citronellal, sowie Mischungen daraus.

Um wahrnehmbar zu sein, muss ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms bzw. Duftstoffs während des Verdampfens, wobei man die Geruchseindrücke in "Kopfnote" (top note), "Herz- bzw. Mittelnote" (middle note bzw. body) sowie "Basisnote" (end note bzw. dry out) unterteilt. Analog zu der Beschreibung in der internationalen Patentveröffentlichung WO 2016/200761 A2 können Kopf-, Herz- und Basisnote anhand ihres Dampfdrucks (mittels der in der WO 2016/200761 beschriebenen Testverfahren bestimmbar) wie nachstehend klassifiziert werden:
Kopfnote: Dampfdruck bei 25°C: >0,0133 kPa
Herznote: Dampfdruck bei 25°C: 0,0133 bis 0,000133 kPa
Basisnote: Dampfdruck bei 25°C: <0,000133 kPa

Zu den haftfesten Riechstoffe, die im Rahmen der vorliegenden Erfindung einsetzbar sind, gehören beispielsweise die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopalvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl.

Höhersiedende bzw. festen Riechstoffe natürlichen oder synthetischen Ursprungs umfassen beispielsweise: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Benzylacetat, Benzylbenzoat, Benzylformiat, Benzylvalerianat, Borneol, Bornylacetat, α-Bromstyrol, n-Decylaldehyd, n-Dodecyl-aldehyd, Eugenol, Eugenolmethylether, Eukalyptol, Farnesol, Fenchon, Fenchylacetat, Geranyl-acetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenol-methylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylaceto-phenon, Methylchavikol, p-Methylchinolin, Methyl-ß-naphthylketon, Methyl-n-nonylacetaldehyd, Methyl-n-nonylketon, Muskon, β-Naphtholethylether, ß-Naphtholmethylether, Nerol, Nitrobenzol, n-Nonylaldehyd, Nonylakohol, n-Octylaldehyd, p-Oxy-Acetophenon, Pentadekanolid, β-Phenyl-ethylakohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimatalkohol, Zimtsäure, Zimtsäureethylester, Zimtsäurebenzylester

Zu den leichter flüchtigen Riechstoffen zählen insbesondere die niedriger siedenden Riechstoffe natürlichen oder synthetischen Ursprungs, die allein oder in Mischungen eingesetzt werden können. Beispiele für leichter flüchtige Riechstoffe sind Alkylisothiocyanate (Alkylsenföle), Butandion, Limonen, Linalool, Linaylacetat und -propionat, Menthol, Menthon, Methyl-n-heptenon, Phellandren, Phenylacetaldehyd, Terpinylacetat, Zitral, Zitronellal.

Bevorzugt einsetzbare Riechstoffverbindungen vom Typ der Aldehyde sind Hydroxycitronellal (CAS 107-75-5), Helional (CAS 1205-17-0), Citral (5392-40-5), Bourgeonal (18127-01-0), Triplal (CAS 27939-60-2), Ligustral (CAS 68039-48-5), Vertocitral (CAS 68039-49-6), Florhydral (CAS 125109-85-5), Citronellal (CAS 106-23-0), Citronellyloxyacetaldehyd (CAS 7492-67-3).

Zusätzlich oder alternativ zu den vorstehend genannten Riechstoffen können auch die in der WO 2016/200761 A2 beschriebenen Riechstoffe, insbesondere die in den Tabellen 1, 2 und 3 genannten Riechstoffe, sowie die in den Tabellen 4a und 4b aufgelisteten Modulatoren eingesetzt werden. Diese Veröffentlichung ist hierin durch Bezugnahme in ihrer Gesamtheit eingeschlossen.

Die erfindungsgemäßen Mikrokapseln können neben Riechstoffen auch andere Öle umfassen. Insbesondere können die Mikrokapseln auch Aktivstoffe in Ölform enthalten, welche für Wasch-, Reinigungs-, Pflege- und/oder Veredelungszwecke geeignet sind, insbesondere
(a) Textilpflegestoffe, wie vorzugsweise Silikonöle, und/oder
(b) Hautpflegestoffe, wie vorzugsweise Vitamin E, natürliche Öle und/oder kosmetische Öle.

Hautpflegende Aktivstoffe sind alle solchen Aktivstoffe die der Haut einen sensorischen und/oder kosmetischen Vorteil verleihen. Hautpflegende Aktivstoffe sind bevorzugt ausgewählt aus den nachfolgenden Substanzen:
a) Wachse wie beispielsweise Carnauba, Spermaceti, Bienenwachs, Lanolin und/oder Derivate derselben und andere.
b) Hydrophobe Pflanzenextrakte
c) Kohlenwasserstoffe wie beispielsweise Squalene und/oder Squalane
d) Höhere Fettsäuren, vorzugsweise solche mit wenigstens 12 Kohlenstoffatomen, beispielsweise Laurinsäure, Stearinsäure, Behensäure, Myristinsäure, Palmitinsäure, Ölsäure, Linolsäure, Linolensäure, Isostearinsäure und/oder mehrfach ungesättigte Fettsäuren und andere.
e) Höhere Fettalkohole, vorzugsweise solche mit wenigstens 12 Kohlenstoffatomen, beispielsweise Laurylalkohol, Cetylalkohol, Stearylalkohol, Oleylalkohol, Behenylalkohol, Cholesterol und/oder 2-Hexadecanaol und andere.
f) Ester, vorzugsweise solche wie Cetyloctanoate, Lauryllactate, Myristyllactate, Cetyllactate, Isopropylmyristate, Myristylmyristate, Isopropylpalmitate, Isopropyladipate, Butylstearate, Decyloleate, Cholesterolisostearate, Glycerolmonostearate, Glyceroldistearate, Glyceroltristearate, Alkyllactate, Alkylcitrate und/oder Alkyltartrate und andere.
g) Lipide wie beispielsweise Cholesterol, Ceramide und/oder Saccharoseester und andere.
h) Vitamine wie beispielsweise die Vitamine A, C und E, Vitaminalkylester, einschließlich Vitamin-C-Alkylester und andere.
i) Sonnenschutzmittel
j) Phospholipide
k) Derivate von alpha-Hydroxysäuren
l) Germizide für den kosmetischen Gebrauch, sowohl synthetische wie beispielsweise Salicylsäure und/oder andere als auch natürliche wie beispielsweise Neemöl und/oder andere.
m) Silikone
n) Natürliche Öle, z.B. Mandelöl
sowie Mischungen jeglicher vorgenannter Komponenten.

In verschiedenen Ausführungsformen enthalten die Mikrokapseln zusätzlich Pflanzenextrakte als Aktivstoff. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel geeignet. Insbesondere bevorzugt sind Extrakte aus Aloe Vera.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein als Aktivstoff mehrere, insbesondere zwei verschiedene Pflanzenextrakten einzusetzen.

Es kann vorteilhaft sein, dass die erfindungsgemäß einsetzbaren äußeren/inneren Mikrokapseln besonders gut auf das behandelte Textil aufziehen. Das wird beispielsweise durch die Verwendung von Aminoplast-Kapseln erreicht, wie solchen auf Melamin-Formaldehyd-Basis. Nach dem Waschprozess weisen insbesondere solche Aminoplast-Kapseln dann üblicherweise eine gewisse Brüchigkeit auf, so dass durch Einwirken mechanischer Kraft eine gezielte Aktivstofffreisetzung, insbesondere Duftfreisetzung, aus der Kapsel stattfinden kann, z. B. beim Abreiben der Haut mit einem Handtuch, welche mit einem entsprechendem Waschmittel gewaschen wurde. Auf diese Weise kann auch nach längerer Lagerung der Wäsche gezielt z. B. ein Wohlgeruch hervorgerufen werden. Dieser gezielt hervorgerufene Wohlgeruch unterscheidet sich von dem Geruch des Produktes, welcher im Wesentlichen durch die herkömmliche Parfümierung bzw. bei der Verwendung diffusiv durchlässiger äußerer Mikrokapseln auch die erste Duftstoffzusammensetzung hervorgerufen wird, da dieser von der aus der inneren Mikrokapsel freigesetzten zweite Duftstoffzusammensetzung, optional in Kombination mit den Resten der gleichzeitig aus den äußeren Mikrokapseln freigesetzten ersten Duftstoffzusammensetzung, dominiert wird. Dadurch wird ein polysensorisches Dufterlebnis ermöglicht, d.h. ein Geruchserlebnis welches für das Produkt als solches charakteristisch ist und bei Öffnen oder der Anwendung auftritt wird im Folgenden durch ein späteres Geruchserlebnis, welches erst nach der Anwendung auftritt abgelöst. Der Verbraucher wird damit in den Stand gesetzt, gezielt Wohlgerüche hervorzurufen, die sich von dem Geruch des Waschmittels unterscheiden.

Insbesondere die Trennung der verschiedenen Duftstoffzusammensetzungen in unterschiedlichen Mikrokapseln bietet den Vorteil eine vergleichsweise scharfe Trennung des Duftempfindens der unterschiedlichen Zusammensetzungen zu ermöglichen und ist bekannten Verfahren, die auf der Mischung unterschiedlich flüchtiger Duftstoffe basieren, weit überlegen.

Die erste und zweite Duftstoffzusammensetzung unterscheiden sich beispielsweise im Hinblick auf ihr Duftprofil und/oder die Flüchtigkeit der enthaltenen Duftstoffe und/oder die Substantivität der enthaltenen Duftstoffe. Es ist insbesondere bevorzugt, dass sich das Duftprofil von erster und zweiter Duftstoffzusammensetzung für den Verbraucher sensorisch wahrnehmbar unterscheidet. Duftprofile können beispielsweise als frisch, grün, ozonig, blumig, Rose, Maiglöckchen, fruchtig, Apfel, Beere, Citrus, holzig, kosmetisch, balsamisch, Amber, Moschus, Fougere oder weiteren beschrieben werden. Zusätzlich oder alternativ können sich die beiden Duftstoffzusammensetzungen in äußerer und innerer Kapsel auch in der physikochemischen Zusammensetzung unterscheiden, d.h. die eingesetzten Duftstoffe/Duftstoffmischungen unterscheiden sich in Zusammensetzung und physikalischen Parametern, wie beispielsweise Dampfdruck, Siedepunkt, Hydrophobie (clogP-Wert), etc.

In verschiedenen Ausführungsformen unterscheiden sich die erste und zweite Duftstoffzusammensetzung dahingehend, dass die erste Duftstoffzusammensetzung mindestens einen Duftstoff, vorzugsweise zwei Duftstoffe, weiter bevorzugt drei Duftstoffe, am bevorzugtesten vier oder mehr Duftstoffe enthält, die in der zweiten Duftstoffzusammensetzung nicht enthalten sind. Ebenso kann es bevorzugt sein, dass die zweite Duftstoffzusammensetzung mindestens einen Duftstoff, vorzugsweise zwei Duftstoffe, weiter bevorzugt drei Duftstoffe, am bevorzugtesten vier oder mehr Duftstoffe enthält, die in der ersten Duftstoffzusammensetzung nicht enthalten sind. Es ist aber nicht ausgeschlossen, dass beide Duftstoffzusammensetzungen die gleichen Duftstoffe enthalten, so lange sich die Zusammensetzung in mindestens einem Duftstoff und/oder den eingesetzten Mengen der Duftstoffe unterscheiden.

Generell ist es in verschiedenen Ausführungsformen bevorzugt, dass die zweite Duftstoffzusammensetzung, d.h. die Zusammensetzung in der inneren Kapsel, höher performant ist als die erste Duftstoffzusammensetzung. Dies hat zur Folge, dass das Geruchsprofil der zweiten Duftstoffzusammensetzung auch bei teilweise gleichzeitiger Freisetzung, beispielsweise wenn durch Reibung Reste der ersten zusammen mit der zweiten Duftstoffzusammensetzung freigesetzt werden, dominiert. Methoden wie dies erreicht werden kann, beispielsweise über die Auswahl von Riechstoffen über ihre Dampfdrücke, sind dem Fachmann bekannt.

Die hierin beschriebenen Mikrokapselsysteme enthalten die Duftstoffzusammensetzungen vorzugsweise in einer Menge von 0,1 bis 95 Gew.-%, bevorzugter 1 bis 90 Gew.-%, noch bevorzugter 5 bis 85 Gew.-% bezogen auf das gesamte Mikrokapselsystem. Dabei macht die erste Duftstoffzusammensetzung vorzugsweise mindestens 30, vorzugsweise mindestens 50 Gew.-%, bis beispielsweise 80 Gew.-%, oder bis 70 Gew.-% der Gesamtmenge an Duftstoffzusammensetzungen in dem Kapselsystem aus. In verschiedenen Ausführungsformen macht das Gewicht der inneren Mikrokapseln bis zu 60 Gew.-%, vorzugsweise 1 bis 50 Gew.-%, noch bevorzugter 2 bis 40 Gew.-% des gesamten Mikrokapselsystems aus. In verschiedenen Ausführungsformen macht das Gewicht des Polymers aus dem die äußere Kapselhülle besteht 1 bis 25 Gew.-%, insbesondere 5 bis 20 Gew.-% des Gesamtgewichts des Mikrokapselsystems aus.

Die Mikrokapselsysteme der vorliegenden Erfindung können in bekannten Formen vorliegen, beispielsweise als Slurry in einem wässrigen Trägermedium oder auch als Pulver.

Bevorzugt enthalten die erfindungsgemäßen Mittel zum Waschen, Reinigen, Konditionieren, Pflegen und/oder Färben von harten oder weichen Oberflächen, Mikrokapseln in Mengen von 0,0001 bis 50 Gew.-%, vorzugsweise 0,01 bis 20 Gew.-%, und insbesondere 0,1 bis 5 Gew.-%, bezogen auf das gesamte Mittel.

Bei den Mitteln zum Waschen, Reinigen, Konditionieren, Pflegen und/oder Färben von harten oder weichen Oberflächen im Sinne dieser Anmeldung handelt es sich um Wasch-, Reinigungs-, Nachbehandlungs- und/oder kosmetische Mittel.

Die erfindungsgemäßen Mittel werden zum Waschen, Reinigen, Konditionieren, Pflegen und/oder Färben von harten oder weichen Oberflächen verwendet. Harte Oberflächen im Sinne dieser Anmeldung sind dabei Fenster, Spiegel und weitere Glasoberflächen, Oberflächen aus Keramik, Kunststoff, Metall oder auch Holz sowie lackiertes Holz, die sich in Haushalt und Gewerbe finden, etwa Badkeramik, Koch- und Speisegeschirr, Küchenoberflächen oder Fußböden. Weiche Oberflächen im Sinne dieser Anmeldung sind textile Flächengebilde, Haut sowie Haare.

Mittel zum Waschen von harten oder weichen Oberflächen im Sinne dieser Anmeldung sind Textilwaschmittel, z.B. in Form von Pulvern, Granulaten, Perlen, Tabletten, Pasten, Gelen, Tüchern, Stücken oder Flüssigkeiten vorliegenden Formulierungen.

Mittel zum Reinigen von harten oder weichen Oberflächen im Sinne dieser Anmeldung umfassen alle Reiniger für harte oder weiche Oberflächen, insbesondere Geschirrspülmittel, Allzweckreiniger, WC-Reiniger, Sanitärreiniger sowie Glasreiniger, Zahncremes, Hautwaschmittel, wie Duschgele, oder Haarwaschmittel.

Mittel zum Konditionieren von harten oder weichen Oberflächen im Sinne dieser Anmeldung sind Weichspüler, Duftspüler, Konditioniertücher für die Anwendung im Wäschetrockner, Hygienespüler, Deodorantien, Antitranspirantien, Haarkonditioniermittel, Stylingmittel und/oder Haarfestigungsmittel.

Mittel zur Pflege von harten oder weichen Oberflächen im Sinne dieser Anmeldung sind Textilpflegemittel, Haarpflegemittel oder Hautbehandlungsmittel, wie beispielsweise Cremes, Lotionen oder Gele.

Mittel zum Färben von harten oder weichen Oberflächen im Sinne dieser Anmeldung sind Haarfärbeund Haartönungsmittel und Mittel zum Aufhellen keratinischer Fasern.

Zusätzlich zu den hierin beschriebenen Kapsel-in-Kapsel-Systemen können die Mittel zusätzliche eine herkömmliche Parfümierung enthalten. Diese unterscheidet sich vorzugsweise sowohl von der ersten als auch der zweiten Duftstoffzusammensetzung der Systeme der Erfindung, beispielsweise in den oben diskutierten Parametern. Diese herkömmliche Parfümierung kann dem Produkt als solchem den eigentlichen Geruch verleihen, welcher beim Öffnen/der Anwendung wahrgenommen wird. Zusätzlich können die Mittel auch weitere herkömmliche Parfümmikrokapseln enthalten, die gleiche oder unterschiedliche Parfümierungen enthalten.

Die Mittel zum Waschen, Reinigen, Konditionieren, Pflegen und/oder Färben von harten oder weichen Oberflächen ermöglichen vorzugsweise die gezielte Freisetzung von Riechstoffen, welche in den äußeren/inneren Kapseln gespeichert sind, weisen aber gleichzeitig einen eigenen Geruch auf, der typischerweise durch eine herkömmliche Parfümierung des Produktes bestimmt wird. Die äußeren/inneren Kapseln sind vorzugsweise innerhalb der Mittel-Matrix stabil und können durch gezielten Reiz, insbesondere mechanische Krafteinwirkung, geöffnet werden, wobei die äußeren Kapseln zusätzlich eine Freisetzung der Riechstoffe mittels Diffusion ermöglichen. Unter mechanische Krafteinwirkung im Sinne der vorliegenden Erfindung wird jede Art der Krafteinwirkung auf die Mikrokapsel verstanden, wie z.B. Scherkräfte, Druck und/oder Reibung. Bei der Anwendung des Mittels, z.B. bei der Textilwäsche oder der Hautreinigung, lagern sich die äußeren Mikrokapseln auf der harten oder weichen Oberfläche ab. Die Freisetzung der Duftstoffe aus den äußeren Mikrokapseln erfolgt dann auf diffusivem Weg, d.h. die Duftstoffe wandern durch das polymere Hüllmaterial und werden dadurch langsam freigesetzt. Nach der Trocknung der Oberfläche können die Mikrokapseln dann z.B. durch Reibung leicht geöffnet werden, wobei die Reibung auch die dadurch aus den äußeren Mikrokapseln freigesetzten inneren Mikrokapseln öffnet. Auf diese Weise gelingt eine gezielte Freisetzung des/der Duftstoff(e) der Reste der ersten Duftstoffzusammensetzung aus den äußeren Kapseln (der Teil, der nicht bereits durch Diffusion freigesetzt wurde) und der zweiten Duftstoffzusammensetzung aus den inneren Kapseln, so dass das Leistungsprofil des gesamten Mittels erhöht wird. Dabei kommt insbesondere der Duftwirkung eine besondere Bedeutung zu, da die Produktleistung in vielen Fällen vom Verbraucher proportional zum Wohlgeruch beurteilt wird.

Die hierin beschriebenen Mittel zum Waschen, Reinigen, Konditionieren, Pflegen und/oder Färben von harten oder weichen Oberflächen ermöglicht eine langanhaltende Duftstofffreisetzung, insbesondere eine langanhaltende Beduftung und Pflege von harten oder weichen Oberflächen (durch die Freisetzung aus der äußeren Mikrokapsel) sowie eine gezielte Duftstofffreisetzung (durch die Freisetzung aus der inneren Mikrokapsel und zusätzlich ggf. auch der äußeren Mikrokapsel), auch nach langen Zeitabständen durch Einsatz der hierin beschriebenen Mikrokapselsysteme.

In einer bevorzugten Ausführungsform handelt es sich bei der Oberfläche um eine textile Oberfläche. Wenn es sich bei der Oberfläche um eine textile Oberfläche handelt ist insbesondere bevorzugt, wenn das Mittel zum Waschen, Reinigen, Konditionieren, Pflegen und/oder Färben von harten oder weichen Oberflächen ein Wasch-, Reinigungs- oder Nachbehandlungsmittel ist.

In einer weiteren Ausführungsform handelt es sich bei der Oberfläche um eine Körperstelle, insbesondere um Haut und/oder Haare. Wenn es sich bei der Oberfläche um eine Körperstelle, insbesondere um Haut und/oder Haare, handelt ist bevorzugt, wenn das Mittel zum Waschen, Reinigen, Konditionieren, Pflegen und/oder Färben von harten oder weichen Oberflächen eine kosmetische Zusammensetzung ist.

Der Kontakt der Mikrokapseln mit der Haut und/oder den Haaren kann entweder durch direkten Kontakt der Haut und/oder den Haaren mit einer kosmetischen Zusammensetzung umfassend Mikrokapseln und/oder durch Übertragung der Mikrokapseln durch Textilien, die solche Mikrokapseln auf der Oberfläche tragen, geschehen.

In den hierin ebenfalls beschriebenen Verfahren zur Herstellung der erfindungsgemäßen Mikrokapselsysteme, werden zuerst die inneren Mikrokapseln, die die zweite Duftstoffzusammensetzung enthalten, mittels im Stand der Technik bekannter Verfahren hergestellt. Diese werden dann mit der ersten Duftstoffzusammensetzung gemischt, vorzugsweise darin dispergiert, und die erhaltene Mischung mittels bekannter Verfahren verkapselt, so dass die äußeren Mikrokapseln gebildet werden.

Die Mikrokapseln, die die zweite Duftstoffzusammensetzung enthalten, können oberflächenmodifiziert sein, um die Verkapselung in der äußeren Mikrokapsel zu ermöglichen. Dazu werden die Mikrokapseln, die die zweite Duftstoffzusammensetzung enthalten (innere Mikrokapseln) beispielsweise mit einem oder mehreren vorzugsweise hydrophoben Modifizierungsmitteln in Wasser suspendiert. Bei den Modifizierungsmitteln kann es sich um mindestens eine Verbindung ausgewählt aus der Gruppe bestehend aus Polyethylenimiden, quartären Ammoniumverbindungen, vorzugsweise solche mit hydrophoben Kohlenwasserstoffresten, quartären Polyvinylpyrrolidonen und ungesättigten Fettsäuren, wie beispielsweise Ölsäure, handeln. Beispiele für geeignete quartäre Ammoniumverbindungen sind Betain, Cholinchlorid, Benzalkoniumchlorid und Di-(C₈₋₈ Alkyl)dimethylammoniumchlorid, wie beispielsweise Didecyldimethylammoniumchlorid. Geeignete Polyethylenimidverbindungen sind multifunktionelle kationische Polymere auf Ethylenimid-Basis mit Molmassen im Bereich von 600 bis 2500000 Da. Solche Polymere sind beispielsweise unter dem Handelsnamen Lupasol von BASF SE kommerziell erhältlich. Genauso sind geeignete quartäre PVPs unter dem Handelsnamen Luviquat von BASF SE kommerziell erhältlich. Generell werden entsprechende Verfahren zur Oberflächenmodifikation und Verkapselung beispielsweise in der EP 2 732 803 A1 für "Sprengmittel" beschrieben und sind ohne Weiteres auf die inneren Mikrokapseln der vorliegenden Erfindung übertragbar. Nach der Modifikation können die Mikrokapseln getrocknet werden, bevor sie dann mit der ersten Duftstoffzusammensetzung kombiniert und in der äußeren Mikrokapsel verkapselt werden.

Es ist selbstverständlich, dass die vorstehend im Kontext mit den erfindungsgemäßen Verfahren beschriebenen oberflächenmodifizierten inneren Mikrokapseln auch in den erfindungsgemäßen Mikrokapselsystemen eingesetzt werden können. In verschiedenen Ausführungsformen der Erfindung sind die inneren Mikrokapseln daher oberflächenmodifizierte Mikrokapseln, wie sie vorstehend beschrieben wurden, insbesondere solche die hydrophob modifiziert wurden, besonders bevorzugt solche, die mit mindestens einem Modifizierungsmittel ausgewählt aus der Gruppe bestehend aus Polyethylenimiden, quartären Ammoniumverbindungen, quartären Polyvinylpyrrolidonen und Ölsäure modifiziert wurden.

Durch die Auswahl und Kontrolle der Reaktionsbedingungen bei der Bildung der Schalen, beispielsweise Kontrolle der Schalendicke, kann deren Durchlässigkeit für die verkapselten Riechstoffe kontrolliert werden. Damit lassen sich beispielsweise innere Mikrokapselschalen erzeugen, die für die verkapselten Riechstoffe undurchlässig sind, und äußere Mikrokapselschalen, die eine Freisetzung mittels Diffusion durch die Kapselschale ermöglichen.

Ebenfalls beschrieben werden Verfahren zur Erzeugung von polysensorischen Dufteindrücken unter Verwendung der hierin beschriebenen Mikrokapselsysteme. In diesen Verfahren erfolgt erst die Freisetzung der ersten Duftstoffzusammensetzung aus der äußeren Mikrokapsel und anschließend zeitlich verzögert die Freisetzung der zweiten Duftstoffzusammensetzung aus der inneren Mikrokapsel. Dadurch kann das Duftprofil abgewandelt und ein polysensorischer Dufteindruck beim Verbraucher erzeugt werden. Das Dufterlebnis kann dadurch erweitert werden, dass in dem Mittel zusätzlich eine herkömmliche, d.h. nicht verkapselte Parfümierung, eingesetzt wird. Dadurch ergibt sich bei der Öffnung und der Anwendung des Produkts ein erster Dufteindruck der im Wesentlichen durch die herkömmliche Parfümierung bewirkt wird, im weiteren Verlauf der Anwendung kommt es zur Freisetzung der ersten Duftstoffzusammensetzung aus den äußeren Mikrokapseln durch Diffusion und schließlich wird durch mechanische Beanspruchung eine Freisetzung der zweiten Duftstoffzusammensetzung aus den inneren Mikrokapseln bewirkt.

In verschiedenen Ausführungsformen dieses Verfahrens ist das Mittel in welchem das hierin beschriebene Kapselsystem eingesetzt wird ein Textilbehandlungsmittel, wie beispielsweise ein Waschmittel oder ein Weichspüler, welches zusätzlich ein herkömmliches Parfüm enthält. Das verwendete Kapselsystem basiert auf Melamin-Formaldehyd-Harz, wobei die äußere Kapsel diffusiv durchlässig und die innere Kapsel geschlossen ist, beide aber aufreibbar sind. In einem ersten Schritt werden bereits vor der Anwendung des Produkts aus diesem die Kopf- und Herznoten der herkömmlichen Parfümierung freigesetzt und sorgen somit für einen ersten Dufteindruck. Nach der Anwendung erhält man ein feuchtes Textil, welches durch die Kopf- und Herznoten der herkömmlichen Parfümierung sowie die diffusiv freigesetzte Duftstoffzusammensetzung aus der äußeren Kapsel olfaktorisch charakterisiert wird. Das trockene Textil zeichnet sich durch die Herz- und Basisnoten der herkömmlichen Parfümierung sowie die diffusiv freigesetzte Duftstoffzusammensetzung aus der äußeren Kapsel aus und nach mechanischer Beanspruchung, beispielsweise durch Reibung oder das Tragen des Textils, kommt die Parfümierung aus der inneren Kapsel dazu bzw. überwiegt. Es ist dabei selbstverständlich, dass im Laufe des Anwendungszyklus das Geruchsprofil des herkömmlichen Parfüms schnell schwächer wird und nach und nach von dem mittels Diffusion freigesetzten Riechstoffen aus der äußeren Mikrokapsel abgelöst wird. Diese werden dann bei mechanischer Beanspruchung, d.h. beim Tragen oder Benutzen des trockenen Textils von den Riechstoffen aus der inneren Mikrokapsel abgelöst.

Auf derartige Art und Weise lassen sich die hierin beschriebenen Mikrokapselsysteme zur Erzeugung polysensorischer Dufteindrücke nutzen.

Die im Zusammenhang mit den erfindungsgemäßen Kapselsystemen beschriebenen Ausführungsformen sind ebenso auf Verfahren zur Herstellung derselben, die diese enthaltenden Mittel sowie die hierin beschriebenen Verwendungen übertragbar und umgekehrt.

Die Erfindung wird im Folgenden anhand von Beispiele illustriert, ist aber nicht auf diese beschränkt.

### Beispiele

### Beispiel 1

Ein handelsüblicher Weichspüler (Vernel conc.) bzw. Waschmittel (Persilpulver) wurde mit einem erfindungsgemäßen Kapselsystem (Muster 2; 0,3 Gew.-% Melamin-Formaldehyd-Kapsel-Slurry: innere Kapsel geschlossen, aufreibbar und beladen mit Parfüm 3, äußere Kapsel diffusiv, aufreibbar und beladen mit Parfüm 2) bzw. einem herkömmlichen Kapselsystem (Muster 1; 0,15 Gew.-% Melamin-Formaldehyd-Kapsel-Slurry: Kapsel geschlossen, aufreibbar und beladen mit Parfüm 3; 0,15 Gew.-% Melamin-Formaldehyd-Kapsel-Slurry: Kapsel diffusiv, aufreibbar und beladen mit Parfüm 2) versetzt und nach Gebrauchsvorschrift beim Waschen von Textilien eingesetzt (Miele Softtronic W1734, Hauptwaschgang bei 40°C, Schleuderzahl bei 1200 rpm, Wasserhärte bei 12° dH, 3,5kg Baumwollfrottiergewebe / Waschgang). Anschließend wurden die Textilien in trockenem Zustand vor und nach Reibung bzw. in feuchtem Zustand gegenüber dem trockenen Zustand von einem Panel geschulter Parfümeure abgerochen und der Duftwechsel auf einer Skala von 1-5 (1=unverändert; 2=leichte Änderung im Duftprofil im Vergleich zur Referenz; 3=mittlere Änderung; 4=starke Änderung; 5=totale Änderung) und die Duftintensität auf einer Skala von 1-10 (1=geruchlos; 10=sehr intensiv) beurteilt.

Beurteilung Duftwechsel trockene Wäsche vor/nach Reibung (nach dem Waschen mit Weichspüler+Mikrokapseln und Trocknen)

| | Trockene Wäsche vor Reibung | Trockene Wäsche nach Reibung |
|---|---|---|
| Muster 1 | Referenz | Referenz |
| Muster 2 | 3,2 | 4,2 |

Es zeigt sich bei der trockenen Wäsche vor der Reibung bereits eine mittlere Änderung des Geruchsprofils im Vergleich zum Referenzsystem, in welchem zwei separate Kapseln eingesetzt werden. Der Grund dafür ist, dass im Referenzsystem auch bei verschiedenen Durchlässigkeiten der Kapselhüllen trotzdem immer beide Duftstoffzusammensetzungen in einem Maße freigesetzt werden, dass der Dufteindruck aus einer Mischung beider verkapselter Zusammensetzungen (d.h. größere Teile aus der diffusiv durchlässigen Kapsel und kleinere Teile aus der im Wesentlichen undurchlässigen Kapsel) resultiert. Demgegenüber ist das erfindungsgemäße Kapsel-in-Kapsel-System vorteilhaft, weil der Geruchseindruck der trockenen Wäsche noch stärker durch die erste Duftstoffzusammensetzung dominiert wird, weil die Freisetzung aus der inneren Kapsel nicht nur durch die weniger durchlässige Kapselwand, sondern auch durch die Verkapselung in der äußeren Mikrokapsel behindert wird. Auf diesen Unterschied ist daher der starke Unterschied zwischen den Geruchsprofilen der beiden getesteten Mikrokapselsysteme zurückzuführen. Dieser Unterschied wird bei der trockenen Wäsche nach der Reibung noch potenziert, indem das Geruchsprofil noch stärker durch die Zusammensetzung in der inneren (der weniger durchlässigen Kapsel) dominiert wird.

Beurteilung Duftintensität trockene Wäsche vor/nach Reibung (nach dem Waschen mit Weichspüler+Mikrokapseln und Trocknen)

| | Trockene Wäsche vor Reibung | Trockene Wäsche nach Reibung |
|---|---|---|
| Muster 1 | 7,6 | 8,0 |
| Muster 2 | 7,9 | 9,2 |

Beurteilung Duftwechsel feuchte Wäsche/trockene Wäsche (nach dem Waschen mit Weichspüler+Mikrokapseln und ggf. Trocknen)

| | Feuchte Wäsche vor Reibung | Trockene Wäsche vor Reibung |
|---|---|---|
| Muster 1 | Referenz | Referenz |
| Muster 2 | 2,6 | 3,2 |

Beurteilung Duftintensität feuchte/trockene Wäsche (nach dem Waschen mit Weichspüler+Mikrokapseln und ggf. Trocknen)

| | Feuchte Wäsche vor Reibung | Trockene Wäsche vor Reibung |
|---|---|---|
| Muster 1 | 8,2 | 7,6 |
| Muster 2 | 8,6 | 7,9 |

Beurteilung Duftwechsel trockene Wäsche vor/nach Reibung (nach dem Waschen mit Waschmittel+Mikrokapseln und Trocknen)

| | Trockene Wäsche vor Reibung | Trockene Wäsche nach Reibung |
|---|---|---|
| Muster 1 | Referenz | Referenz |
| Muster 2 | 2,8 | 3,4 |

Beurteilung Duftintensität trockene Wäsche vor/nach Reibung (nach dem Waschen mit Waschmittel+Mikrokapseln und Trocknen)

| | Trockene Wäsche vor Reibung | Trockene Wäsche nach Reibung |
|---|---|---|
| Muster 1 | 6,4 | 7,6 |
| Muster 2 | 7,2 | 8,2 |

Wie bereits oben im Detail diskutiert, ist generell ist bei Muster 2 eine deutlichere Trennschärfe der Duftprofile vor und nach Reibung sowie bei feuchter und trockener Wäsche erkennbar.

## Patentansprüche

1. Mikrokapselsystem umfassend eine äußere Mikrokapsel mit einer äußeren Kapselhülle, wobei die äußere Mikrokapsel enthält:
(a) mindestens eine darin eingeschlossene innere Mikrokapsel mit einer inneren Kapselhülle; und
(b) eine erste Duftstoffzusammensetzung;
wobei die Kapselhülle der äußeren Mikrokapsel die innere Mikrokapsel und die erste Duftstoffzusammensetzung vollständig umgibt,
**dadurch gekennzeichnet, dass**
die innere Mikrokapsel eine zweite Duftstoffzusammensetzung enthält, welche vollständig von der inneren Kapselhülle der inneren Mikrokapsel umgeben ist und sich von der ersten Duftstoffzusammensetzung unterscheidet,
und dass die äußere Kapselhülle zumindest für Teile der ersten Duftstoffzusammensetzung partiell durchlässig ist und diese durch Diffusion freigesetzt werden.

2. Mikrokapselsystem nach Anspruch 1, **dadurch gekennzeichnet, dass** die äußere Mikrokapsel durchschnittlich mehr als eine, vorzugsweise mindestens 2, noch bevorzugter mindestens 3, am bevorzugtesten 4 oder mehr darin eingeschlossene innere Mikrokapseln enthält.

3. Mikrokapselsystem nach einem der vorangehenden Ansprüche, **dadurch gekennzeichnet, dass** sich die äußere Kapselhülle der äußeren Mikrokapsel und die innere Kapselhülle der inneren Mikrokapsel in ihrem Freisetzungsverhalten unterscheiden.

4. Mikrokapselsystem nach Anspruch 3, **dadurch gekennzeichnet, dass** die innere Kapselhülle für die zweite Duftstoffzusammensetzung weniger durchlässig als die äußere Kapselhülle für die erste Duftstoffzusammensetzung ist.

5. Mikrokapselsystem nach Anspruch 3 oder 4, **dadurch gekennzeichnet, dass** die Freisetzung der Duftstoffzusammensetzung aus der inneren und optional auch der äußeren Mikrokapsel durch mechanische Beanspruchung erfolgt.

6. Mikrokapselsystem nach einem der vorangehenden Ansprüche, **dadurch gekennzeichnet, dass** sich die erste und zweite Duftstoffzusammensetzung im Hinblick auf ihr Duftprofil und/oder die physikochemischen Eigenschaften unterscheiden.

7. Verfahren zur Herstellung eines Mikrokapselsystems nach einem der Ansprüche 1-6, **dadurch gekennzeichnet, dass** das Verfahren umfasst (1) Bereitstellen von Mikrokapseln enthaltend die zweite Duftstoffzusammensetzung und einer ersten Duftstoffzusammensetzung, (2) Verkapseln der Mikrokapseln, die die zweite Duftstoffzusammensetzung enthalten, und der ersten Duftstoffzusammensetzung in einer äußeren Mikrokapsel.

8. Mittel zum Waschen, Reinigen, Konditionieren, Pflegen und/oder Färben von harten oder weichen Oberflächen enthaltend das Mikrokapselsystem nach einem der Ansprüche 1-6.

9. Verwendung des Mikrokapselsystems nach einem der Ansprüche 1-6 zur Erzeugung polysensorischer Dufteindrücke.

## Claims

1. A microcapsule system comprising an outer microcapsule having an outer capsule shell, the outer microcapsule containing:
(a) at least one inner microcapsule enclosed therein having an inner capsule shell; and
(b) a first fragrance composition;
wherein the capsule shell of the outer microcapsule completely surrounds the inner microcapsule and the first fragrance composition,
**characterized in that**
the inner microcapsule contains a second fragrance composition which is completely surrounded by the inner capsule shell of the inner microcapsule and is different from the first fragrance composition,
and **in that** the outer capsule shell is at least partially permeable to parts of the first fragrance composition and said parts are released by diffusion.

2. The microcapsule system according to claim 1, **characterized in that** the outer microcapsule contains on average more than one, preferably at least two, more preferably at least three, most preferably four or more, inner microcapsules enclosed therein.

3. The microcapsule system according to any one of the preceding claims, **characterized in that** the outer capsule shell of the outer microcapsule and the inner capsule shell of the inner microcapsule differ in terms of their release behavior.

4. The microcapsule system according to claim 3, **characterized in that** the inner capsule shell is less permeable to the second fragrance composition than the outer capsule shell is to the first fragrance composition.

5. The microcapsule system according to claim 3 or 4, **characterized in that** the fragrance composition is released from the inner capsule and optionally also from the outer microcapsule under mechanical stress.

6. The microcapsule system according to any one of the preceding claims, **characterized in that** the first fragrance composition and the second fragrance composition differ with regard to their fragrance profile and/or physicochemical properties.

7. A method for producing a microcapsule system according to any one of claims 1-6, **characterized in that** the method comprises (1) providing microcapsules containing the second fragrance composition, and a first fragrance composition, (2) encapsulating the microcapsules containing the second fragrance composition, and the first fragrance composition, in an outer microcapsule.

8. An agent for washing, cleaning, conditioning, caring for and/or dyeing hard or soft surfaces, containing the microcapsule system according to any one of claims 1-6.

9. The use of the microcapsule system according to any one of claims 1-6 for producing polysensory fragrance impressions.

## Revendications

1. Système de microcapsules comprenant une microcapsule externe comportant une enveloppe de capsule externe, la microcapsule externe contenant :
(a) au moins une microcapsule interne renfermée à l'intérieur et comportant une enveloppe de capsule interne ; et
(b) une première composition parfumée ;
dans lequel l'enveloppe de capsule de la microcapsule externe entoure complètement la microcapsule interne et la première composition parfumée,
**caractérisé en ce que**
la microcapsule interne contient une seconde composition parfumée qui est complètement entourée par l'enveloppe de capsule interne de la microcapsule interne et qui diffère de la première composition parfumée,
et que l'enveloppe de capsule externe est partiellement perméable à au moins des parties de la première composition parfumée et lesdites parties sont libérées par diffusion.

2. Système de microcapsules selon la revendication 1, **caractérisé en ce que** la microcapsule externe contient en moyenne plus d'une, de préférence au moins deux, plus préférablement au moins trois, le plus préférablement quatre ou plus microcapsules internes renfermées à l'intérieur.

3. Système de microcapsules selon l'une des revendications précédentes, **caractérisé en ce que** l'enveloppe de capsule externe de la microcapsule externe et l'enveloppe de capsule interne de la microcapsule interne diffèrent par leur comportement de libération.

4. Système de microcapsules selon la revendication 3, **caractérisé en ce que** l'enveloppe de capsule interne est moins perméable pour la seconde composition parfumée que l'enveloppe de capsule externe pour la première composition parfumée.

5. Système de microcapsules selon la revendication 3 ou 4, **caractérisé en ce que** la composition parfumée est libérée de la microcapsule interne et éventuellement également de la microcapsule externe par contrainte mécanique.

6. Système de microcapsules selon l'une des revendications précédentes, **caractérisé en ce que** les première et seconde compositions parfumées diffèrent en ce qui concerne leur profil parfumé et/ou les propriétés physico-chimiques.

7. Procédé de production d'un système de microcapsules selon l'une des revendications 1 à 6, **caractérisé en ce que** le procédé comprend (1) la fourniture de microcapsules contenant la seconde composition parfumée et une première composition parfumée, (2) l'encapsulation des microcapsules, lesquelles contiennent la seconde composition parfumée, et de la première composition parfumée dans une microcapsule externe.

8. Agent de lavage, de nettoyage, de conditionnement, d'entretien et/ou de coloration de surfaces dures ou molles contenant le système de microcapsules selon l'une des revendications 1 à 6.

9. Utilisation du système de microcapsules selon l'une des revendications 1 à 6 pour produire des impressions parfumées polysensorielles.
